# NEW EUROPEAN PATENT SPECIFICATION

(11) **EP 0 324 938 B2**
(45) Date of publication and mention of the opposition decision: **27.02.2008**
(45) Mention of the grant of the patent: 18.11.1993
(21) Application number: 88120371.5
(22) Date of filing: 06.12.1988
(51) Int. Cl.: A61K 49/00, A61K 9/50, G01N 29/04

(54) **Concentrated stabilized microbubble-type ultrasonic imaging agent and method of production**
Konzentriertes und stabilisiertes Ultraschallagens vom Mikrobubbletyp zur Bilderzeugung und Herstellungsverfahren
Agent ultrasonique d'imagerie de type microbulle concentré et stabilisé et son mode de préparation

(30) Priority: 29.12.1987 US 139576; 29.12.1987 US 139577
(43) Date of publication of application: 26.07.1989
(73) Proprietor: GE Healthcare AS, 0401 Oslo (NO)
(72) Inventor: Widder, Kenneth J., Del Mar, CA 92014 (US); Westkaemper, Peter J., San Diego, CA 91109 (US)
(74) Representative: Wright, Simon Mark

(56) References cited:
- EP-A- 0 224 934
- Keller et al. (1987), Am. Heart J. 114, 570-575

## Description

This invention relates to ultrasonic imaging agents and methods for production thereof.

It has been known since 1968-70 that contrast echo-cardiography can be used to delineate intracardiac structures, assess valvular competence, demonstrate intracardiac shunts, and identify pericardial effusion. (Gramiak and Shah, 1968; and Feigenbaum, et al., 1970.) Ultrasonic imaging of the heart potentially has important advantages of convenience, safety, and reduced cost over present diagnostic procedures, such as angiography, which requires the use of radio-opaque dyes for X-ray imaging, or the use of radio-nuclide imaging agents for radio-imaging However, progress in practical applications of ultrasonic imaging has been delayed by the lack of effective clinically-usable imaging agents.

Ultrasonic imaging utilizes an ultrasonic scanner to generate and receive sound waves. The scanner is placed on a body surface overlying the area to be imaged, and sound waves are directed toward that area. The scanner detects reflected sound waves and translates that data into images. When ultrasonic energy is transmitted through a substance, the acoustic properties of the substance depend upon the velocity of the transmission and the density of the substance. Changes in the substance's acoustic properties (e.g., variations in acoustic impedence) are most prominent at the interfaces of different substances, such as a liquid-solid or liquid-gas interface. Consequently, when ultrasonic energy is directed through media, changes in acoustic properties will result in more intense sound reflection signals for detection by the ultrasonic scanner.

Ultrasonic imaging agents can consist of small solid or gaseous particles which, when injected in the circulatory system, provide improved sound reflection and image clarity. Microbubble-type imaging agents consist of minute bubbles of a gas (usually air) which are dispersed in a carrier liquid for parenteral injection. The "microbubbles" are carried by the circulatory system to the organ being imaged.

It has been proposed to form a dispersion of air microbubbles in a warm aqueous gelatin solution, and cooling the solution to a solidification temperature to trap the microbubbles. For administration, the gelled dispersion is to be warned until it liquifies, and parenterally administered with the microbubbles dispersed in the liquified gelatin. (Tickner, et al. U.S. Patent 4,276,885; and Tickner, et al., National Technical Information Service Report HR-62917-1A, April, 1977).

Gelatin-trapped microbubbles on introduction into the bloodstream have a short life-time. They rapidly dissapear. Another disadvantage is that the microbubbles are too large to pass through capillary beds, and are therefore not suitable for heart imaging by peripheral intravenous administration.

The discovery by Dr. Steven B. Feinstein of sonication-produced microbubble imaging agents represented an important advance in this art. Using viscous aqueous solutions, such as 70% sorbitol or dextrose, Dr. Feinstein produced a dispersion of microbubbles by high energy sonication of the solutions. The resulting microbubbles had sizes less than 10 µm, and were capable of passing through capillary beds. The persistence of the microbubbles, although of the order of a few minutes, permitted the imaging agent to be prepared and administered intravenously for heart imaging. (Feinstein, et al., 1984; and Feinstein U.S. Patent 4,572,203.)

Subsequently, Dr. Feinstein sought to improve the persistence of the microbubbles. He found that by sonication of a heat-sensitive protein, such as albumin, microbubbles of improved stability were obtained. (See Feinstein, PCT Application WO 84/02838, corresponding to allowed U.S. application Serial No. 805,975, filed December 5, 1985). Concentrations of microbubbles of 10 to 14 x 10⁶ microbubbles per ml were obtained with bubble sizes from 2 to 9 µm (Keller, Feinstein, and Watson, 1987). The microbubbles persisted for 24 to 48 h.

However, the sonication-produced albumin microbubble imaging agent of Feinstein was not sufficiently stable for commercial manufacture. Stabilities of the order of weeks or months (rather than hours or days) are required to permit an imaging agent to be manufactured at a central location and distributed to hospitals in the United States and other countries. For commercially feasible manufacture, shipment and hospital storage prior to use, a stability time of at least four weeks is needed and preferably at least eight weeks or longer.

Further, for the most effective imaging, it is desirable to have the highest obtainable concentration of microbubbles in the imaging agent. But the population of microbubbles of the desired small sizes tends to decrease with holding of the sonicated albumin solutions. The small bubble size attrition can occur either by collapse of the microbubbles, or by coalesence to oversize microbubbles. Consequently a further important objective has been to find means for increasing concentrations of microbubbles in the imaging agent. An imaging agent of very high microbubble concentration is inherently better, and a safety factor is provided. With a concentration of microbubbles higher than the minimum required for effective imaging, some loss of the microbubbles of the desired size can be accepted.

The present invention provides a concentrated, room-temperature stable ultrasonic imaging agent comprising a parenterally administrable aqueous medium containing a dispersion of microspheres at least 80% of which have diameters in the range of 1 to 9 *µ*m, said microspheres consisting of gas microbubbles encapsulated in a water-insolubilized heat-denaturable biocompatible protein, and being suspended in an aqueous solution of the same protein in which the microbubbles are encapsulated, said imaging agent having a homogeneously dispersed concentration of from 300 to 600 x 10⁶ microspheres per ml and maintaining this concentration for at least 8 weeks at a temperature of 20 to 25°C. In optimized embodiments, microsphere concentrations of the order of 300 to 500 x 10⁶ microspheres per ml are achieved. Surprisingly, these ultra-high concentrations can be maintained for over eight weeks. The imaging agents of this invention are therefore adapted for manufacture and distribution on a commercial basis. Following shipment, they may be maintained in inventory by hospitals for many weeks, being available for diagnostic use as required.

The imaging agents of this invention are produced from a heat-denaturable biocompatible protein by a step-wise sonication procedure. As with the Feinstein method, an aqueous solution of protein is subjected to sonication to form gas microbubbles while concurrently heating the solution to insolubilize small portions of the protein. However, the improved sonication procedure, which results in the increased concentration of highly stable microbubbles utilizes a novel sequential sonication. In the initial sonication phase, the sonicator horn is directly contacted with the solution (viz. by immersion just below the upper suface of the solution). This initial sonication is carried out without appreciable foaming of the solution. In the next phase of the sonication, foaming is promoted. The sonicator horn is withdrawn to a position in the ambient atmosphere above but proximate to the surface of the solution. Intense foaming and aerosolating occurs. The population of microbubbles is thereby greatly increased and the microbubbles are encapsulated with denatured protein to obtain a dispersion of highly stable microspheres. Moreover, the stability of the microspheres permits them to be concentrated and/or fractionated. By such manipulations, bubble concentration can be doubled or tripled and oversize bubbles eliminated.

For example, the concentration of the microspheres as initially produced can be from 50 to 150 x 10⁶. By a float separation concentration procedure, the microsphere concentration can be increased 200 to 600 x 10⁶ microspheres per ml. Also, by another float-type separation, most of the microbubbles of larger size than 10 µm can be removed, resulting in an imaging agent composed predominately of microspheres of diameters substantially less than 10 µm. At least 80% of the microspheres have diameters in the range from 1 to 9 µm.

The accompanying drawings illustrate a preferred method of preparing the ultrasound imaging agent of this invention.

FIGS. 1A to 1D illustrate the steps in the sequential sonication procedure.

FIG. 2 is a cross-sectional view taken on line 2-2 of FIG. 1B, illustrating the relation of the sonicator horn to the inside of the syringe which contains the albumin solution being sonicated.

FIG. 3 illustrates a separator vessel in which increments of the microsphere dispersions are pooled for float separation concentration.

FIGS. 4, 4A, and 4B illustrate a method of fractionation of microsphere dispersions to remove oversize microspheres.

FIG. 5 is a graph of experimental data showing the concentration of the microspheres in the imaging agent as produced, and their storage stability.

The starting material for practicing this invention is an aqueous solution of a suitable biocompatible material. The encapsulating material should be heat-sensitive so that it can be partially insolubilized by heating during sonication. More specifically, coincident with the sonication, a small portion of the dissolved biocompatible material is heated or otherwise treated so that its solubility is reduced. This results in a small volume of solid phase material, which forms the encapsulating layers around the microspheres. A heat-sensitive protein is selected such as albumin, hemoglobin, collagen, etc. For administration to humans, human protein is preferred. Human serum albumin (HSA) is especially suitable. HSA is available commercially as a sterile 5% aqueous solution, which can be used directly as the starting material for preparing the microspheres. However, other concentrations of albumin or other heat-denaturable proteins can be used. HSA concentration can be varied, for example, within the range from 1 to 25% by weight.

Commercially-available sonicator equipment may be used in practicing this invention. Theoretically, sonicator vibration frequencies can vary over a considerable range, such as from 5 to 30 kHz, but most commercially-available sonicators operate at 20 kHz or 10 kHz. The 20 kHz sonicators perform well for the purpose of this invention. Such sonicator equipment can be obtained from Heat Systems-Ultrasonics, Inc., Farmingdale, New York, and other companies. Ultrasonics Model W-380 or similar model can be used with a flat tip, high gain sonicator horn. The power applied to the sonicator horn can be varied over power settings scaled from 1 to 10 by the manufacturer, as with Ultrasonics Model W-380. An intermediate power setting can be used (viz. from 4 to 8). The vibrational frequency and the power applied must be sufficient to produce cavitation in the liquid being sonicated.

The solution to be sonicated can be treated in small increments. For example, 8 ml quantities of the solution can be individually sonicated. Initial sonication can be carried out with the flat-ended sonicator horn in contact with the solution, preferably immersed in the upper portion of the solution. Immersion is desirable in order to carry out the initial sonication without appreciable foaming. With a power setting of 4 to 6, the initial sonication can be performed in less than a min (viz. 15 to 45 s).

Immediately following the initial phase of the sonication, the sonicator horn is withdrawn to a position above the solution but proximate to the upper surface of the solution. In the second phase, the sonication is deliberately carried out in such manner as to produce intense foaming of the solution, contrary to conventional sonications, where it is desirable to avoid foaming. For the purpose of the present invention, foaming and aerosolating are important for obtaining the imaging agent of enhanced concentration and stability.

To promote foaming, the power input to the sonicator horn may be increased in the second stage. For example, the power setting may be moved from an initial setting of 4 to a setting of 6. The second phase of the sonication can be carried out in less than a min, (viz. from 15 to 45 s). The total time for the sonication for both the first and second phases can be of the order of one min. For example, a 25 to 35 s sonication can be used for each phase. The foaming produced in the second phase of the sonication is immediately detectable by the cloudy appearance of the solution, and by the foam produced.

By means of the sequential sonication, comprising the cavitation phase followed by a foaming phase, the concentration of the encapsulated microbubbles, referred to herein as "microspheres", can be greatly increased. Concentrations in excess of 25 x 10⁶ microspheres per ml are easily obtainable, such as from 50 to 150 x 10⁶ concentrations. Moreover, the resulting microspheres will be predominantly of diameters less than 10 µm. 80% or more of the microspheres have diameters in the range from 1 to 9 µm with a mean diameter of 4 to 6 µm.

When the sonication is carried out in contact with air as the ambient atmosphere, the microspheres will have air centers. Air is believed to be the most convenient ambient atmosphere, but, if desired, sonication could be carried out under other gas atmospheres (viz. nitrogen, oxygen, carbon dioxide, etc.).

Following initial production, the microsphere dispersions can be further processed to increase the concentration and/or to remove oversize microspheres. Since the microspheres are buoyant they tend to rise to the surface of the dispersion. By holding the dispersion without agitation for a number of hours, (viz. for 4 to 12 h), most of the microspheres will rise to the surface and concentrate in an upper layer above the clarified solution. By this "float-separation" of the microspheres into an upper layer, portions of the clarified solution can be removed from below the microspheres, thereby obtaining a dispersion of greater microsphere concentration. For example, from 50 to 75% of the solution volume may be removed in this concentration process.

Either before or after the above-described concentration, float-separation of oversized microspheres can be obtained. Large size microspheres such as one having diameters greater than 10 µm have relatively greater buoyancy. They will therefore rise more rapidly to the surface of the solution. By utilizing a short holding time, such as from 15 to 45 min, the largest size microspheres can be selectively collected in a small upper layer above a dispersion which will still contain substantially all of the microspheres of small size. By removing this microsphere dispersion from beneath the layer of oversize microspheres, a fractionation may be achieved in which the larger microspheres will remain in the vessel in which the fractionation is carried out.

The imaging agent produced by this combination of two-stage sonication and the float-separation concentration can have a homogeneously-dispersed concentration of greater than 300 x 10⁶, such as from 300 to 900 x 10⁶ (3 to 9 x 10⁸) microspheres per ml. High concentrations can be maintained for long periods of holding at ambient room temperatures (20-25 °C). Concentrations above 200 and typically above 300 x 10⁶ microspheres per ml can be maintained for periods of at least four and usually eight weeks or longer.

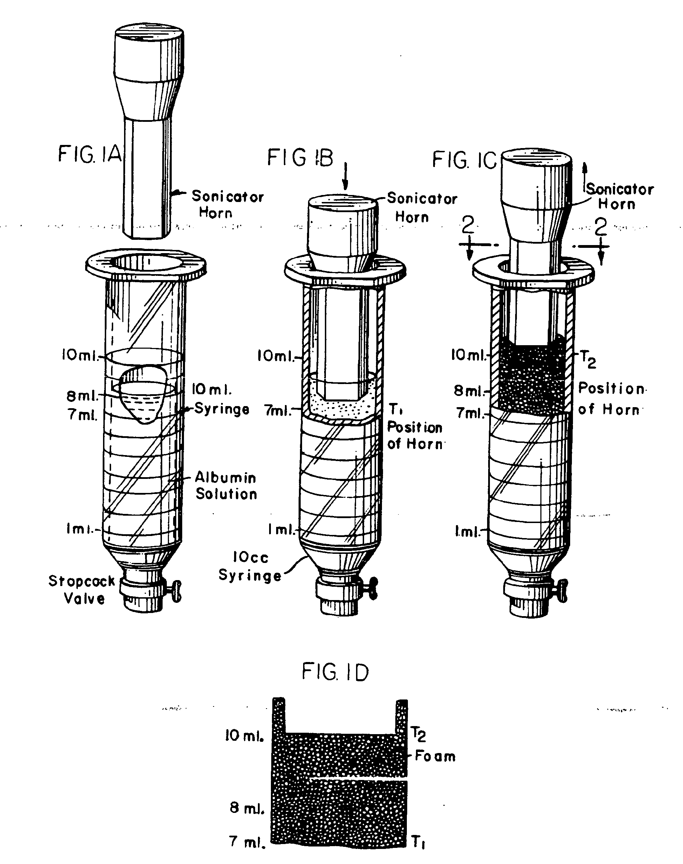

In FIG. 1A, there is shown a 10 ml syringe having an open top and a stopcock-type valve at its lower discharge end. The syringe is filled to the 8 ml level with the 5% albumin (HSA) solution. The sonicator horn is inserted in the syringe to the 7 ml level, indicated as the T₁ position in FIG. 1B. In this position, the sonicator horn is immersed in the upper portion of the solution, the solution level being as indicated in FIG. 1B. Initial sonication is carried out essentially without foaming of the solution.

Immediately following initial sonication and without turning off the sonicator, the horn is withdrawn to the 10 ml level, indicated as the T₂ position in FIG. 1C. The power input to the sonicator horn can also be increased as it is withdrawn to the T₂ position. Immediately following the withdrawal, foaming of the albumin solution commences and the solution becomes milky in appearance. The solution will foam upwardly around the sonicator horn during the second phase. The appearance of the foamed solution is illustrated in FIG. 1D, the microbubbles being indicated in greatly enlarged diameter over their actual micron range sizes.

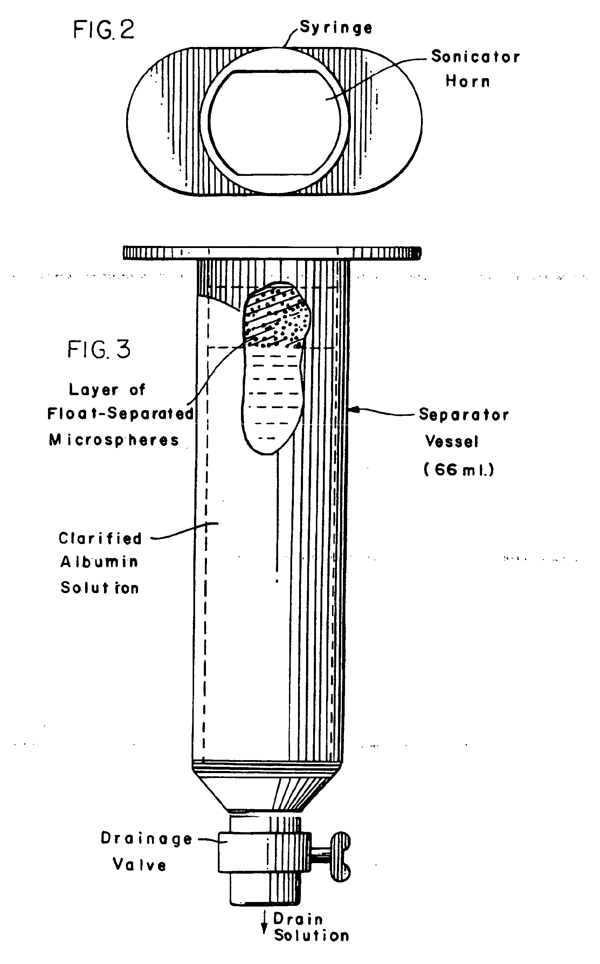

The solution being sonicated contains both dissolved and entrained air. The solution is in contact with the ambient atmostphere around the sonicator horn. (The clearance between the horn and the inside of the syringe can be seen in the cross-sectional view of FIG. 2.) The air contact facilitates the foaming and aerosolating of the solution in the second stage of the sonication.

The dispersions from a plurality of sonication batches can be pooled for concentration. For example, a plurality of the dispersion increments can be introduced into a separator vessel, which may be a large syringe or separator funnel equipped at its bottom with an outlet controlled by a drainage valve. Such a separate vessel in the form of a large syringe is shown in FIG. 3. By holding the pooled dispersions for several hours without agitation, such as overnight holding, the microspheres will rise to the top of the solution and form a layer of float-separated microspheres. Beneath the collected layer, the clarified albumin solution will be substantially free of microspheres. It is therefore possible to drain off a major portion of the solution through the bottom outlet. For example, one-half to three-fourths of the solution can be removed. However, it is desirable to retain a sufficient solution volume to permit full redispersion of the concentrated microspheres.

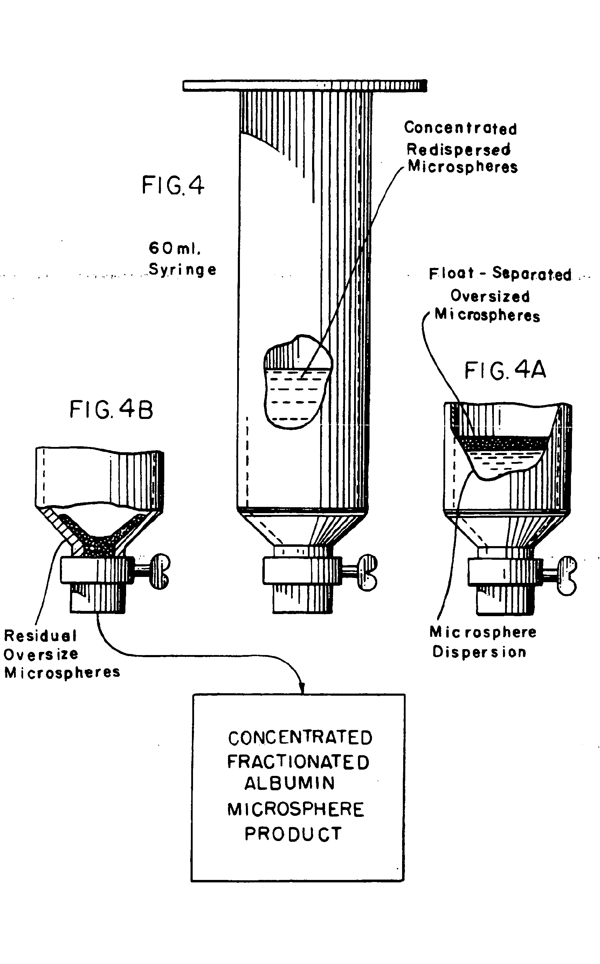

In FIG. 4 illustrates the microsphere concentrate with the microspheres redispersed. The microspheres are sufficiently stable that they do not adhere permanently to each other in a concentrated layer, remaining as separate intact microspheres. They can readily be redispersed by mild agitation.

After redispersion to an essentially homogeneous condition, fractionation may be carried out to remove oversize microspheres. By holding the redispersion for a short time, such as around 30 min, the largest diameter microspheres will preferentially rise to the top and collect in a layer, as indicated in FIG. 4A. When that has occurred, the microsphere dispersion beneath the oversize microspheres can be removed through the drainage valve. When the collected oversize microspheres approach the valve, the valve is closed so that the oversize layer remains in the separator vessel, as indicated in FIG. 4B. The product obtained is a concentrated fractionated albumin microsphere product in which at least 80% of the microspheres have diameters in the range from 1 to 9 µm. The preferred product has at least 90% of the microspheres with diameters of from 2 to 8 µm.

Further directional details of the presently preferred procedures are set out below under the appropriate headings.

### Sonication:

Fill a 10 ml syringe of oval cross-section fitted at its lower outlet end with a stopcock to the 8 ml mark with sterile 5% human serum albumin. Position a sonicator probe of smaller cross-section in the syringe so that the bottom of the probe is at the 7 ml mark. Sonicate at energy setting 6 for 30 s then (with the sonicator still on) move the probe tip to the 10 ml mark, while moving the energy setting to 8. Sonicate for an additional 25 s. Turn off sonicator, remove probe and drain contents of the syringe into a 60 ml syringe or separatory funnel with a stopcock controlled bottom outlet. From 5 to 6 syringe volumes are pooled.

### Concentration:

Allow the pooled increments to stand overnight (8-12 h ) without agitation in the separator vessel. When substantially all the microspheres have formed a layer on the top, drain two-thirds of the volume from the bottom.

### Fractionation:

Resuspend the microspheres and fill a 60 ml syringe with them. Let sit 30 min, then drain all but about the last 3-4 ml into a collection vessel. The oversize microspheres are left. Count a sample and calculate the concentration, mean diameter, and percentage less than 10 µm. If less than 99.5% are less than 10 µm, re-fractionate. If required for redispersion, concentration may be adjusted with 5% HSA.

### RESULTS

Concentration measurements are set out below in Table A for three representative runs using the procedures described above. The initial concentration of the disperions after sonication was of the order of 130 to 140 x 10⁶/ml. This was increased by the float-separation concentration to 340 to 450 x 10⁶/ml.

For product control, the microspheres may be counted by a Coulter Counter, obtainable from Coulter Electronics, Inc., Highleah, Florida (viz. Coulter Counter Model TAII). Microsphere counts set out above were determined in this way.

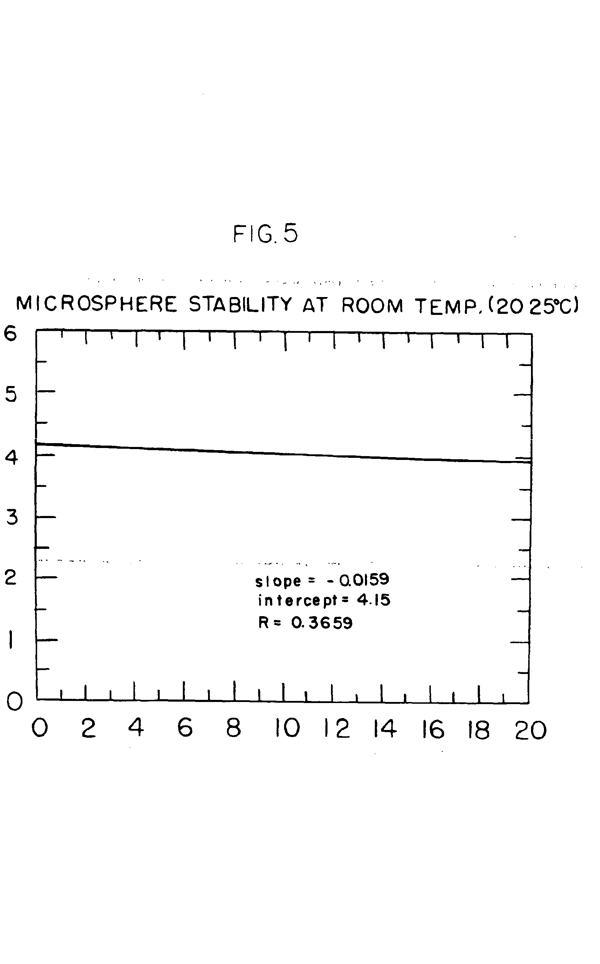

The stability of a representative product was examined in a study lasting for 20 weeks. The initial concentration was approximately 4.31 x 10⁸ (431 x 10⁶) microspheres per ml. Concentration measurements were made at about weekly intervals. The results are summarized in Table B. The measurements, which were made by means of a Coulter Counter, are presented graphically in FIG. 5. The samples were held at ambient room temperature (20-25°C). The concentration of about 400 x10⁶ microspheres per ml was maintained for 20 weeks. This evidences a high degree of room temperature stability.

The stability of the microspheres can be affected by unusually hot or cold temperatures. However, even at temperatures as low as 4°C or as high as 37°C, microsphere concentrations in excess of 200 x 10⁶/ml can be maintained for periods of eight weeks or longer. Nevertheless, for commercial distribution or long-term holding very high or low temperatures should be avoided. Room temperature holding is preferred. Temperature protection of the microspheres during shipment can be used.

**TABLE A**

| Concentration Measurements | | |
|---|---|---|
| Runs | Microspheres/ml After Sonication | Microspheres/ml After Concentration |
| A | 135 x 10⁶ | 386 x 10⁶ |
| B | 141 x 10⁶ | 483 x 10⁶ |
| C | 133 x 10⁶ | 440 x 10⁶ |

**TABLE B**

| Week | Microsphere Concentration x 10⁸ |
|---|---|
| 0 | 4.31 |
| 1 | 4.49 |
| 2 | 4.20 |
| 3 | 3.91 |
| 4 | 3.86 |
| 5 | 4.25 |
| 6 | 4.06 |
| 7 | 4.12 |
| 8 | 3.92 |
| 9 | 3.94 |
| 10 | 3.97 |
| 11 | 3.48 |
| 12 | 3.48 |
| 13 | 4.09 |
| 14 | 3.70 |
| 15 | 4.92 |
| 17 | 4.15 |
| 18 | 3.99 |
| 19 | 4.14 |

### REFERENCES

Feigenbaum, et al. (1970), Circulation 41:615-621
Feinstein, U.S. Patent 4,572,203.
Feinstein PCT Application WO 84/02838.
Feinstein, et al. (1984), J. Am. Coll. Cardiol. 3:14-20.
   Gramiak and Shah (1968), Invest. Radiol. 3:356-358.
   Keller, Feinstein and Watson (1987), Amer. Heart J., 114:570-575.
   Tickner et al. U.S. Patent 4,276,885.
   Tickner et al., National Technical Information Service Report HR 62917-1A, April, 1977, pages 34-40.

## Claims

1. A concentrated room-tamperatbre stable ultrasonic imaging agent comprising a parenterally administerable aqueous medium containing a dispersion of microspheres at least 80% of which have diameters in the range of 1 to 9 µm, said microspheres consisting of gas microbubbles encapsulated in a water-insolubilized heat-denaturable biocompatible protein, and being suspended in an aqueous solution of the same protein in which the microbubbles are encapsulated, said imaging agent having a homogeneously dispersed concentration of from 300 to 600 x 10⁶ microspheres per ml, and which maintains such concentration for at least 8 weeks at a temperature of 20 to 25° C.

2. The imaging agent of claim 1 in which said microbubbles are encapsulated with human serum albumin.

3. The imaging agent of claim 1 in which 90% or more of said microspheres have diameters in the range from 2 to 8 µm.

4. A concentrated room-temperature stable ultrasonic imaging agent for intravenous administration, comprising a sterile aqueous solution of human serum albumin containing a dispersion of microspheres at least 80% of which have diameters in the range of 1 to 9 µm, said microspheres consisting of a bubble of air encapsulated In a water-insolubilized layer of said albumin. said imaging agent having a homogeneously-dispersed concentration of from 300 to 600 x 10⁶ microspheres per ml and maintaining this concentration for at least 8 weeks al a temperature of 20 to 25° C.

5. The imaging agent of claim 4 in which at least 90% of said microspheres have diameters in the range of 2 to 8 µm.

6. A method of producing a dispersion of microspheres according to any of claims 1 to 5 for use as an ultrasonic imaging agent in which an aqueous solution of a heal-denaturable biocompatible protein is subjected to sonication to form gas microbubbles while heating said solution to insolubilize a portion of the protein, wherein the improvement comprises: during an initial sonication phase directly contacting the tip of the sonicator horn with said solution and carrying out the sonication and heating of said solution without appreciable foaming, then withdrawing the sonicator horn to a position in the ambient atmosphere proximate to the surface of said solution and foaming the solution to increase the population of microbubbles, and encapsulating the microbubbles with denatured protein to obtain a dispersion of stabilized microspheres of increased concentration

7. The method of claim 6 in which said protein is human serum albumin

8. A method of producing a concentrated dispersion of microspheres for use as an ultrasonic imaging agent in which a solution of human serum albumin is subjected to a sonication process according to claim 6, wherein the improvement comprises holding a body of the thus-obtained microsphere dispersion without agitation for sufficient time to permit the microspheres to rise therein and concentrate in an upper layer above the clarified albumin solution, and separating a portion of the clarified albumin solution from the concentrated layer to obtain a dispersion of greater microsphere concentration.

9. The method of claim 8 in which said dispersion of greater concentration is fractionated by withdrawing a dispersion containing most of the microspheres while leaving behind a microsphere fraction containing the largest size microspheres which had collected near the upper surface of the dispersion.

10. The method of claim 8 in which at least 80% of said microspheres have diameters in the range from 1 to 9 µm but include microspheres of larger diameters, which method includes the further steps of holding a body of the microsphere dispersions without agitation for sufficient time to permit at least the microspheres of larger than 10 µm diameter to rise therein and concentrate in an upper layer, and withdrawing a dispersion from beneath said upper layer containing most of the microspheres of less man 10 µm diameter while leaving behind a microsphere fraction containing most of the microspheres of larger than 10 µm diameter.

## Patentansprüche

1. Konzentriertes, bei Raumtemperatur stabiles Ultraschallabbildungsmittel, umfassend ein parenteral verabreichbares wässriges Medium, welches eine Dispersion von Mikrokügelchen enthält, von denen mindestens 80% Durchmesser im Bereich von 1 bis 9 µm haben, wobei die Mikrokügelchen aus Gasmikrobläschen bestehen, die in einem wasserunlöslich gemachten wärmedenaturierbaren bioverträglichen Protein eingekapselt und in einer wässrigen Lösung des gleichen Proteins, in dem die Mikrobläschen eingekapselt sind, suspendiert sind, wobei das Abbildungsmittel eine homogen dispergierte Konzentration von 300 bis 600 x 10⁶ Mikrokügelchen pro Milliliter hat und eine solche Konzentration mindestens 8 Wochen lang bei einer Temperatur von 20 bis 25°C aufrecht erhält.

2. Abbildungsmittel nach Anspruch 1, in welchem die Mikrobläschen mit menschlichem Serumalbumin eingekapselt sind.

3. Abbildungsmittel nach Anspruch 1, in welchem 90% oder mehr der Mikrokügelchen Durchmesser im Bereich von 2 bis 8 µm haben.

4. Konzentriertes, bei Raumtemperatur stabiles Ultraschallabbildungsmittel zur intravenösen Verabreichung, umfassend eine sterile wässrige Lösung von menschlichem Serumalbumin, welche eine Dispersion von Mikrokügelchen enthält, von denen mindestens 80% Durchmesser im Bereich von 1 bis 9 µm haben, wobei die Mikrokügelchen aus einer Luftblase bestehen, die in einer wasserunlöslich gemachten Schicht aus dem Albumin eingekapselt sind, wobei das Abbildungsmittel eine homogen dispergierte Konzentration von 300 bis 600 x 10⁶ Mikrokügelchen pro Milliliter hat und diese Konzentration mindestens 8 Wochen lang bei einer Temperatur von 20 bis 25°C aufrecht erhält.

5. Abbildungsmittel nach Anspruch 4, in welchem mindestens 90% der Mikrokügelchen Durchmesser im Bereich von 2 bis 8 µm haben.

6. Verfahren zum Herstellen einer Dispersion von Mikrokügelchen nach einem der Ansprüche 1 bis 5 zur Verwendung als Ultraschallabbildungsmittel, in welchem eine wässrige Lösung eines wärmedenaturierbaren bioverträglichen Proteins einer Beschallung ausgesetzt wird, um Gasmikrobläschen zu bilden, während die Lösung erwärmt wird, um einen Teil des Proteins unlöslich zu machen, wobei die Verbesserung umfasst: das direkte Inkontaktbringen der Spitze des schallerzeugenden Horns mit der Lösung während einer anfänglichen Beschallungsphase und das Durchführen der Beschallung und Erwärmen der Lösung ohne nennenswertes Schäumen der Lösung, dann das Zurückziehen des schallerzeugenden Horns in eine Position in der umgebenden Atmosphäre in der Nähe der Oberfläche der Lösung und Schäumen der Lösung, um die Anzahl der Mikrobläschen zu vergrößern, und das Einkapseln der Mikrobläschen mit denaturiertem Protein, um eine Dispersion von stabilisierten Mikrokügelchen mit einer erhöhten Konzentration zu erhalten.

7. Verfahren nach Anspruch 6, in welchem das Protein menschliches Serumalbumin ist.

8. Verfahren zum Herstellen einer konzentrierten Dispersion von Mikrokügelchen zur Verwendung als Ultraschallabbildungsmittel, in welchem eine wässrige Lösung von menschlichem Serumalbumin einem Beschallungsverfahren nach Anspruch 6 ausgesetzt wird, worin die Verbesserung umfasst das Halten eines Volumens der so erhaltenen Mikrokügelchendispersion ohne Rühren während eines ausreichenden Zeitraums, um den Mikrokügelchen zu ermöglichen, darin aufzusteigen und sich in einer oberen Schicht oberhalb der geklärten Albuminlösung zu konzentrieren, und das Abtrennen eines Teils der geklärten Albuminlösung von der konzentrierten Schicht, um eine Dispersion mit größerer Mikrokügelchenkonzentration zu erhalten.

9. Verfahren nach Anspruch 8, in welchem die Dispersion mit größerer Konzentration fraktioniert wird, indem eine Dispersion abgezogen wird, die die meisten der Mikrokügelchen enthält, während eine Mikrokügelchenfraktion zurückgelassen wird, welche die Mikrokügelchen mit der größten Größe enthält, welche sich nahe der Oberfläche der Dispersion angesammelt hatte.

10. Verfahren nach Anspruch 8, in dem mindestens 80% der Mikrokügelchen Durchmesser im Bereich von 1 bis 9 µm haben, aber Mikrokügelchen mit größeren Durchmessern einschließen, wobei das Verfahren die weiteren Schritte des Haltens eines Volumens der Mikrokügelchendispersion ohne Rühren während eines ausreichenden Zeitraums, um mindestens den Mikrokügelchen mit mehr als 10 µm Durchmesser zu ermöglichen, darin aufzusteigen und sich in einer oberen Schicht zu konzentrieren, und das Abziehen einer Dispersion von unterhalb dieser oberen Schicht, welche die meisten der Mikrokügelchen mit weniger als 10 µm Durchmesser enthält, wobei eine Mikrokügelchenfraktion zurückgelassen wird, welche die meisten der Mikrokügelchen mit mehr als 10 µm Durchmesser enthält, einschließt.

## Revendications

1. Agent pour imagerie ultrasonore concentré et stable à la température ambiante comprenant un milieu aqueux pouvant être administré par voie parentérale contenant une dispersion de microsphères dont au moins 80% ont des diamètres dans l'intervalle de 1 à 9 µm, ces microsphères étant constituées de microbulles de gaz encapsulées dans une protéine biocompatible insolubilisée à l'eau et pouvant être dénaturée à la chaleur, et étant en suspension dans une solution aqueuse de la même protéine que celle dans laquelle sont encapsulées les microbulles, cet agent pour imagerie ayant une concentration dispersée de façon homogène de 300 à 600 x 10⁶ microsphères par ml et conservant cette concentration pendant au moins 8 semaines à une température de 20 à 25°C.

2. Agent pour imagerie selon la revendication 1, dans lequel lesdites microbulles sont encapsulées avec de la sérumalbumine humaine.

3. Agent pour imagerie selon la revendication 1, dans lequel 90% ou plus desdites microsphères ont des diamètres dans l'intervalle de 2 à 8 µm.

4. Agent pour imagerie ultrasonore concentré et stable à la température ambiante pour administration par voie intraveineuse, comprenant une solution aqueuse stérile de sérumalbumine humaine contenant une dispersion de microsphères dont au moins 80% ont des diamètres dans l'intervalle de 1 à 9 µm, ces microsphères étant constituées d'une bulle d'air encapsulée dans une couche de ladite albumine insolubilisée à l'eau, ledit agent pour imagerie ayant une concentration dispersée de façon homogène de 300 à 600 x 10⁶ microsphères par ml et conservant cette concentration pendant au moins 8 semaines à une température de 20 à 25 °C.

5. Agent pour imagerie selon la revendication 4, dans lequel au moins 90% desdites microsphères ont des diamètres dans l'intervalle de 2 à 8 *µ*m.

6. Procédé pour produire une dispersion de microsphères selon l'une quelconque des revendications 1 à 5 pour une utilisation comme agent pour imagerie ultrasonore, dans lequel une solution aqueuse d'une protéine biocompatible pouvant être dénaturée à la chaleur est soumise à des sons pour former des microbulles de gaz tout en chauffant ladite solution pour insolubiliser une partie de la protéine, l'amélioration consistant à: lors d'une phase initiale d'exposition aux sons, mettre directement en contact la pointe du cornet émetteur de sons avec ladite solution et effectuer l'exposition aux sons et le chauffage de ladite solution sans moussage notable, retirer le cornet émetteur de sons jusqu'à une position située dans l'atmosphère ambiante, au voisinage de la surface de ladite solution, faire mousser la solution pour accroître la population de microbulles, puis encapsuler les microbulles avec une protéine dénaturée afin d'obtenir une dispersion présentant une plus grande concentration en microsphères stabilisées.

7. Procédé selon la revendication 6, dans lequel ladite protéine est la sérumalbumine humaine.

8. Procédé pour produire une dispersion concentrée de microsphères pour une utilisation comme agent pour imagerie ultrasonore, dans lequel une solution de sérumalbumine humaine est soumise à des sons selon le procédé de la revendication 6, l'amélioration consistant à maintenir un volume de la dispersion de microsphères ainsi obtenue sans agitation pendant un temps suffisant pour permettre aux microsphères de monter dans celle-ci et de se concentrer dans une couche supérieure au-dessus de la solution d'albumine clarifiée, et à séparer une partie de la solution d'albumine clarifiée de la couche concentrée afin d'obtenir une dispersion présentant une plus grande concentration en microsphères.

9. Procédé selon la revendication 8, dans lequel ladite dispersion de concentration plus élevée est fractionnée par extraction d'une dispersion contenant la majeure partie des microsphères tout en laissant une fraction de microsphères contenant les microsphères de plus grandes tailles qui se sont accumulées au voisinage de la surface supérieure de la dispersion.

10. Procédé selon la revendication 8, dans lequel au moins 80% desdites microsphères ont des diamètres dans l'intervalle de 1 à 9 *µ*m, mais incluent des microsphères de diamètres plus grands, lequel procédé comporte les étapes supplémentaires consistant à maintenir un volume des dispersions de microsphères sans agitation pendant un temps suffisant pour permettre au moins aux microsphères d'un diamètre supérieur à 10 *µ*m de monter dans celles-ci et de se concentrer dans une couche supérieure, et à extraire une dispersion par le bas de cette couche supérieure, qui contient la majeure partie des microsphères d'un diamètre inférieur à 10 *µ*m, tout en laissant une fraction de microsphères contenant la majeure partie des microsphères d'un diamètre supérieur à 10 µm.
